# EUROPEAN PATENT SPECIFICATION

(11) **EP 2 505 136 B1**
(45) Date of publication and mention of the grant of the patent: **14.06.2017**
(21) Application number: 09851500.0
(22) Date of filing: 25.11.2009
(51) Int. Cl.: A61B 5/053, A61B 5/04

(54) **ELECTRODE APPARATUS FOR MEASURING IMPEDANCE WITHIN THE HUMAN BODY, AND APPARATUS USING SAME FOR MEASURING IMPEDANCE WITHIN THE HUMAN BODY AND PERFORMING TREATMENT**
ELEKTRODENGERÄT ZUR IMPEDANZMESSUNG IM MENSCHLICHEN KÖRPER SOWIE VORRICHTUNG DAMIT ZUR IMPEDANZMESSUNG IM MENSCHLICHEN KÖRPER UND ZUR DURCHFÜHRUNG EINER BEHANDLUNG
DISPOSITIF D'ÉLECTRODE POUR MESURER L'IMPÉDANCE À L'INTÉRIEUR DU CORPS HUMAIN, ET APPAREIL UTILISANT UN TEL DISPOSITIF À POUR MESURER L'IMPÉDANCE À L'INTÉRIEUR DU CORPS HUMAIN ET EFFECTUER UN TRAITEMENT

(30) Priority: 23.11.2009 KR 20090113118
(43) Date of publication of application: 03.10.2012
(73) Proprietor: MSP Co., Ltd, Incheon 404-834 (KR)
(72) Inventor: PARK, Moon Seo, Incheon 403-010 (KR)
(74) Representative: Hoefer & Partner Patentanwälte mbB
(86) International application number: PCT/KR2009/006953
(87) International publication number: WO 2011/062315

(56) References cited:
- GB-A- 2 115 700
- GB-A- 2 318 642
- KR-A- 20050 072 965
- US-A1- 2006 085 049
- US-A1- 2009 156 925
- US-B1- 6 181 974
- US-B1- 6 280 454

## Description

### TECHNICAL FIELD

The present disclosure relates to an apparatus for measuring impedance of a subject including human and treating certain conditions of the subject. More particularly, it relates to an apparatus for measuring impedance of a human body and treating a certain condition of a human body, which can precisely and automatically locate acupuncture points of a human body, display the location as a three-dimensional image, and facilitate acupuncture treatment using the located/displayed actupuncture points.

### BACKGROUND ART

Acupuncture treats certain conditions of a human body (e.g., brain) by physically (e.g., mechanically or electrically) stimulating acupuncture points of the human body to change certain functions of the human body. The acupuncture's treating effects vary depending on whether the acupuncture points are precisely located. Practitioners locate the acupuncture points by their past experience and personal technique.

Electrical Impedance Tomography (EIT) was recently proposed and has been used as a medical imaging technique for some reasons. First, hardware systems using EIT are not expensive. Second, EIT is non-destructive and safe to a subject (e.g., human body). Third, temporal resolution provided by EIT is greater than those provided by X-ray or MRI while the spatial resolution of retrieved images is lower.

Acupuncture points of a human body are two-dimensional anatomically. Different human bodies have different distribution patterns of nerve tissues and soft tissues. Locating acupuncture points of a human body thus tends to rely on medical professionals' experience. EIT analyzes acupuncture points of a human body from the perspective of engineering.

According to EIT, a few milivolts of electric current at 10 to 100kHz are allowed to flow through a human body and a resistance of the human body in response to the electric current is measured. Acupuncture points of a human body have an impedance lower than that of tissues surrounding the acupuncture points. To find out the electrical characteristics of cross-sections of a human body, a plurality of electrodes are attached to a human body, a certain level of electric current is allowed to flow through the human body in a predetermined order, resistance data in response to the electric current is measured, and the resistance data is displayed as an image.

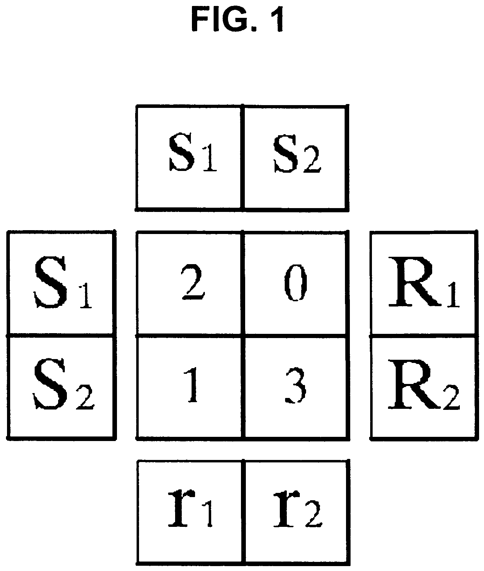

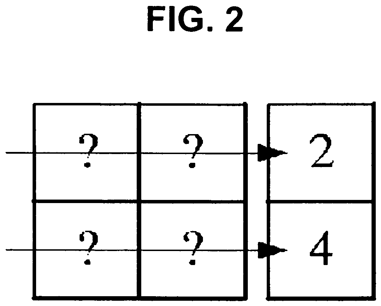

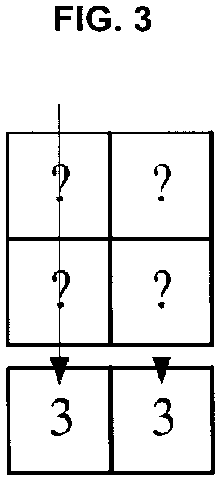

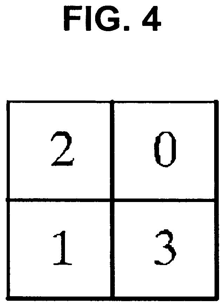

A prior art electrical impedance tomography is described with reference to the drawings. FIGS. 1 to 4 show the principle of imaging resistance data of a human body in the prior art electrical impedance tomography.

A plurality of electrodes are attached to a human body. Electric current is allowed to flow through the human body sequentially. Resulting resistance data is measured and displayed as an image. As shown in FIG. 1, for example, 2 x 2 of input electrodes (S, s) and receiving electrodes (R, r) are attached to a human body, electric current is allowed to flow, and resulting resistance is measured.

In detail, as shown in FIG. 1, parallel input electrodes (S1, S2), parallel receiving electrodes (R1, R2), vertical input electrodes (s1, s2), and vertical receiving electrodes (r1, r2) are attached to a subject. As shown in FIG. 2, electric current is then allowed to flow from the parallel input electrodes (S1, S2) to the parallel receiving electrodes (R1, R2) and impedance in the horizontal direction is measured. As shown in FIG. 3, electric current is allowed to flow from the vertical input electrodes (s1, s2) to the vertical receiving electrodes (r1, r2) and impedance in the vertical direction is measured. As a result, as shown in FIG. 4, distribution of impedances of a human body can be estimated by reverse non-linear data processing.

Typically, an EIT apparatus has cylindrical electrodes that are designed to be attached to the skin of a human body by, for example, surrounding the skin (e.g., ankle, wrist, and the like). After the electrodes are attached to the skin, electrical current is allowed to flow through the human body sequentially and resulting resistances are measured. For example, the measured vertical and horizontal resistance values are the sum of all resistances of the body. Distribution of resistance data with regard to a certain cross-section of the body can be detected. Alternatively, from a known resistance distribution data, voltage distribution can be calculated according to the strength of the current, thereby being able to display an equipotential line.

The prior art EIT apparatus, however, has the following problems.

First, as skeletons of a human body are imaged, it takes a lont time to perform data processing.

Second, depending on how the electrodes are attached to a human body, resolution of the image of acupuncture points can be seginificantly low.

Third, although acupuncture points can be real-time detected, acupunctural treatment cannot be performed using the detected points.

Prior art document GB 2 115 700 A concerns a contact electrode for electronic instruments wherein within a cylindrical outer electrode an insulating insert and a hollow guide bush are provided. Within the guide bush a needle electrode is slidingly seated. One end of the needle electrode with a contact surface protrudes from the outer cylindrical electrode and the guide bush to the outside of the device. The other end of the needle electrode is mechanically and conductively connected to a spring within the outer electrode.

Prior art document US 2009/0156925 A1 discloses an active sensor module for the measurement of bioelectricity. The sensor module comprises an active electrode which is inserted in an opening of a cap and which abuts by means of a protrusion at an inner surface of the cap. The electrode is biased by a spring which is situated between an end of the electrode opposing the contact surface and an inner wall of a main body.

Prior art document GB 2 318 642 A describes a self adjusting electrode for sensing galvanic skin resistance. A combination of planar electrodes is mounted on a plate which abuts on an inner surface of a housing in a region of an opening in the housing. The plate and therefore the electrodes are biased by a spring which forces the plate against the abutment surface of the housing.

Document US 6,181,974 B1 concerns a facial contact electrode wherein the electrodes is formed by his crew screwed into a cylindrical housing which resides between an assembly of inner and outer liners.

### DETAILED DESCRIPTION OF THE INVENTION

### TECHNICAL PROBLEM

It is an object underlying the present invention to provide an electrode device for measuring impedances of a human body and an apparatus for measuring the impedance of a human body which allow a more reliable and less time consuming measurement with a higher accuracy.

To solve the above-described problems, the present invention provides an electrode device for measuring impedances of a human body and an apparatus comprising the electrode device. The electrode device and the apparatus can automatically and precisely locate acupuncture points (meridians) of a human body, display the points as a three-dimensional image, and allow a precise acupuncture treatment to be performed at a desired location.

### TECHNICAL SOLUTION

The objects underlying the present invention are achieved by an electrode device according to independent claim 1 and by an apparatuses according to independent claims 6 and 15. Preferred embodiments are defined and the respect of dependent claims.

In one aspect, the present invention provides an electrode device for measuring impedances of a human body, comprising: a cylindrical housing member having a guide rod mounted to an open side thereof; a cylindrical electrode member configured to be pressed against the skin of a human body and moved back and forth through the open side along the guide rod; and a resilient member interposed between the cylindrical electrode member and the cylindrical housing member to resiliently move the cylindrical electrode member back and forth along the guide rod.

In some embodiments, the cylindrical electrode member is an electrode made of a conductive material or an electrode coated with a conductive material. The conductive material may be, e.g., gold.

In some embodiments, a protruding portion is formed at the open end of the cylindrical electrode member such that engagement between the cylindrical electrode member and the cylindrical housing member is facilitated.

In some embodiments, the cylindrical housing member is made of a conductive material or an electrode coated with a conductive material.

In some embodiments, a first housing protruding portion is formed at the open side of the cylindrical housing member and a second housing protruding portion is formed at the other side of the cylindrical housing member.

In some embodiments, the resilient member is made of a conductive material. The resilient member may be a spring, for example.

In another aspect, the present invention provides an apparatus for measuring impedance of a human body, which comprises: a base plate having a plurality of electrode holes formed in a lattice structure and a plurality of a first needle holes formed in a lattice structure such that the electrode holes and the first needle holes are arranged alternately; a plurality of electrodes mounted to the electrode holes; and first and second electrical lines connected to the electrodes.

In some embodiments, the distance between two adjacent electrode holes and the distance between two adjacent first needle holes are set in a range of 5mm to 20mm.

In some embodiments, the apparatus may further comprise a cover configured to be engaged with the base plate to protect the electrodes and the first and second electrical lines. Preferably, the cover may include a plurality of second needle holes arranged such that the respective second needle holes can be positioned over the respective first needle holes of the base plate when the cover is engaged with the base plate. Preferably, the outer width of the second needle holes may be designed to be greater than the inner width thereof.

In some embodiments, the cover is provided with at least one identifying member. Preferably, the cover may be made of silicone.

In some embodiments, the cover may be shaped to be a hemisphere or a cylinder. Preferably, the cover may further comprise a plurality of guide hole members each connecting the first needle holes and the second needle holes.

In some embodiments, the first electrical lines are formed between the respective electrodes and the respective first needle holes in the direction of X-axis and the second electrical lines are formed between the respective electrodes and the respective first needle holes in the direction of Y-axis.

In some embodiments, the first electrical lines are connected to input electrodes and the second electrical lines are connected to receiving electrodes.

In another aspect, the present inventions provides an apparatus for measuring impedances of a human body, comprising: a circular base plate including a plurality of electrode hoes and a plurality of first needle holes, the electrode holes and the first needle holes being formed eccentrically and alternately; a plurality of electrodes mounted to the electrode holes; and first and second electrical lines connected to the electrodes. Preferably, the first electrical lines and the second electrical lines may be connected to the electrodes alternately.

In still another aspect, the present invention provides an apparatus for measuring impedances of a human body, comprising: a base plate having a plurality of parts that are provided radially with a predetermined distance between two adjacent parts, each of which parts has a plurality of electrode holes and a plurality of first needle holes, the electrode holes and the first needle holes being mounted alternately; a plurality of electrodes mounted to the electrode holes; and first and second electrical lines connected to the electrode holes alternately. Preferably, the first electrical lines may be connected to input electrodes and the second electrical lines are connected to receiving electrodes.

In yet another aspect, the present invention provides an apparatus for measuring impedances of a human body, comprising: a base plate having a plurality of parts that are provided radially with a predetermined distance between two adjacent parts, each of which parts has a plurality of electrode holes and a plurality of first needle holes, the electrode holes and the first needle holes being mounted alternately with respect to the center of base plate; a plurality of electrode devices mounted to the electrode holes; and first and second electrical lines connected to the electrode holes alternately. Preferably, the first electrical lines may be connected to input electrodes and the second electrical lines are connected to receiving electrodes.

In a further aspect, the present invention provides an apparatus for measuring impedances of a human body, comprising: a base plate having a plurality of radial holes provided radially with a predetermined distance between two adjacent holes, the base plate having a plurality of electrode holes and a plurality of first needle holes between two adjacent radial holes; a plurality of electrode devices mounted to the electrode holes; and first and second electrical lines connected to the electrode devices alternately, wherein the electrode holes and the first needle holes are mounted alternately with respect to the center of base plate. Preferably, the first electrical lines may be connected to input electrodes and the second electrical lines are connected to receiving electrodes.

### ADVANTAGEOUS EFFECT

The present invention provides advantageous effects including the following. First, the data about the location of acupuncture points can be easily and quickly obtained. Second, acupuncture points can be located precisely regardless of curvature of the skin of a human body. Third, acupuncture points can be located and displayed without decrease in resolution regardless of the skin to be contacted by the apparatus. Fourth, the data about acupuncture points can be displayed as a three-dimensional image in combination with CT or MRI devices. Fifth, acupuncture points can be located automatically and acupuncture treatment can be performed according to the located points, which allow a user even without sufficient experience to use the apparatus.

### BRIEF DESCRIPTION OF THE DRAWINGS

FIGS. 1 to 4 illustrate the principle of displaying resistance of a human body as an image according to a conventional electrical impedance tomography.

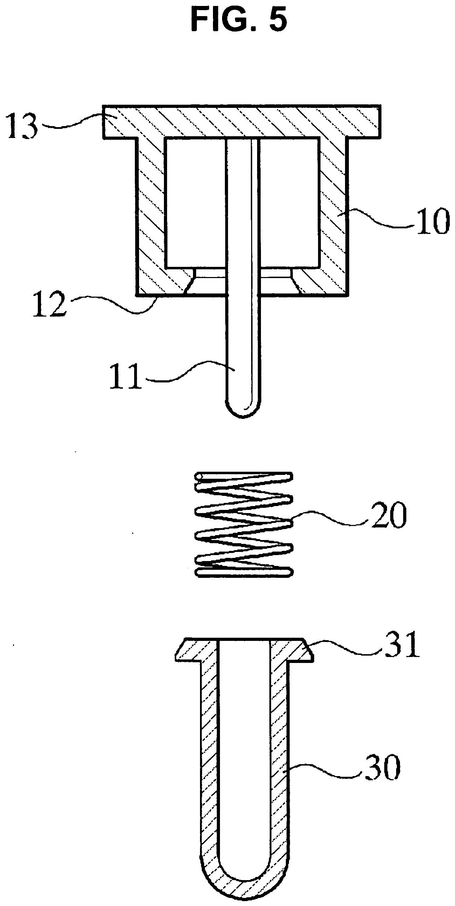

FIG. 5 is a cross-sectional view showing a dissembled state of an electrode device for measuring impedance of a human body according to the first embodiment of the present invention.

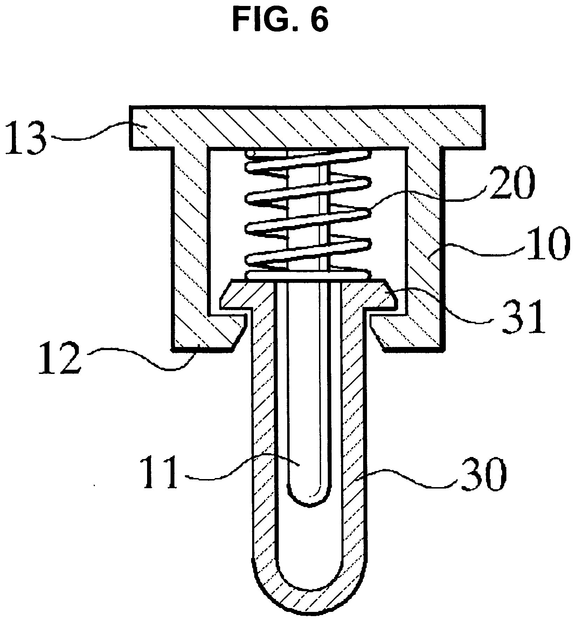

FIG. 6 is a cross-sectional view showing an assembled state of the electrode device of FIG. 5.

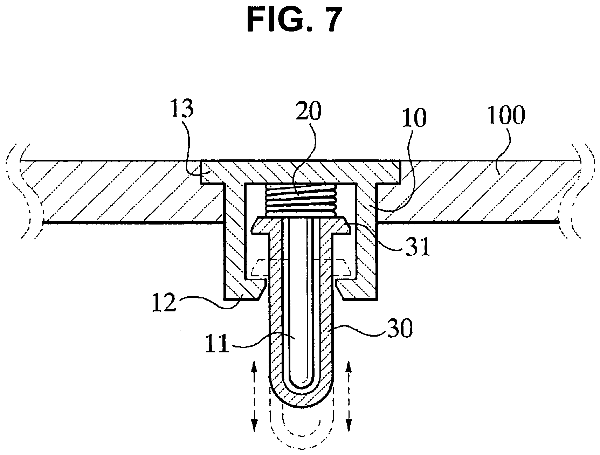

FIG. 7 is a cross-sectional view showing an example of application of the electrode device of FIG. 5.

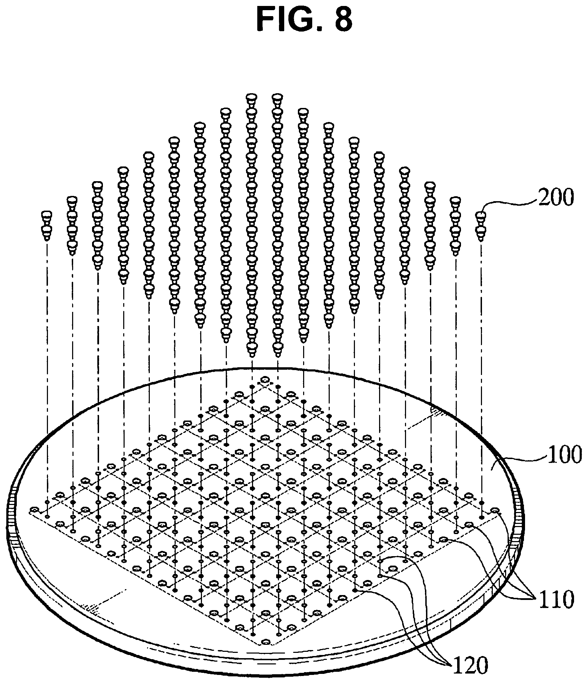

FIG. 8 is a perspective view of an apparatus for measuring impedance of a human body according to the first embodiment of the present invention.

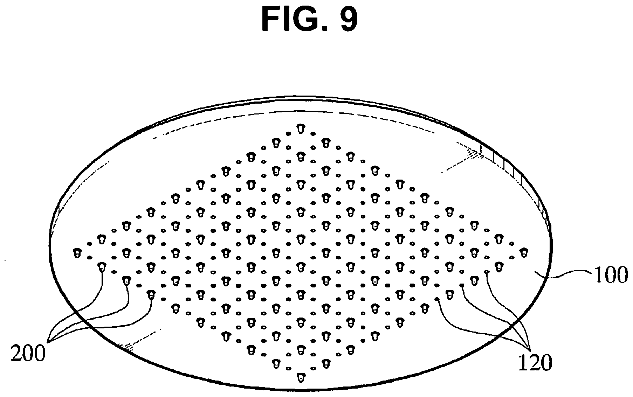

FIG. 9 is a perspective view of the bottom of the apparatus of FIG. 8.

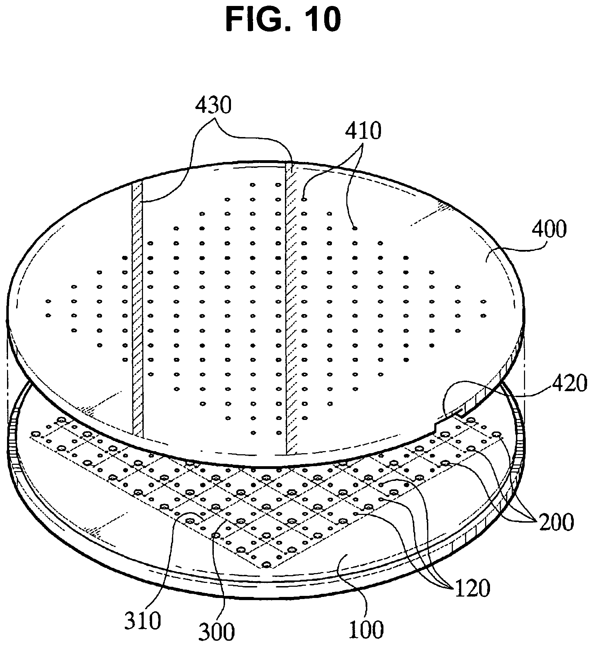

FIG. 10 is a perspective view showing the apparatus of FIG. 8 comprising an an examplary cover.

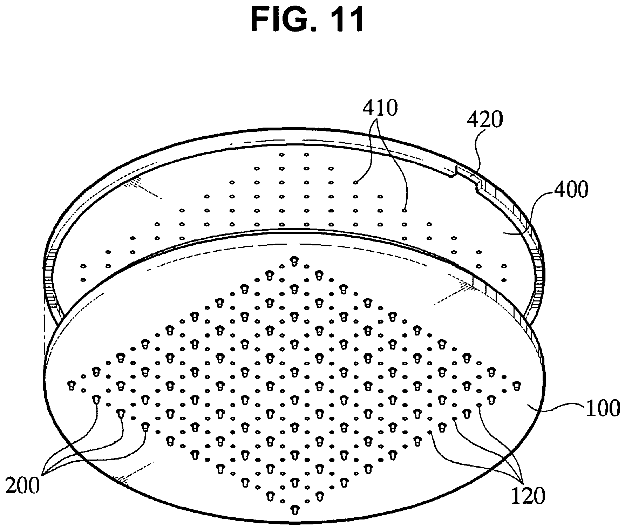

FIG. 11 is a perspective view showing the bottom of the the apparatus of FIG. 8 comprsing an exemplary cover.

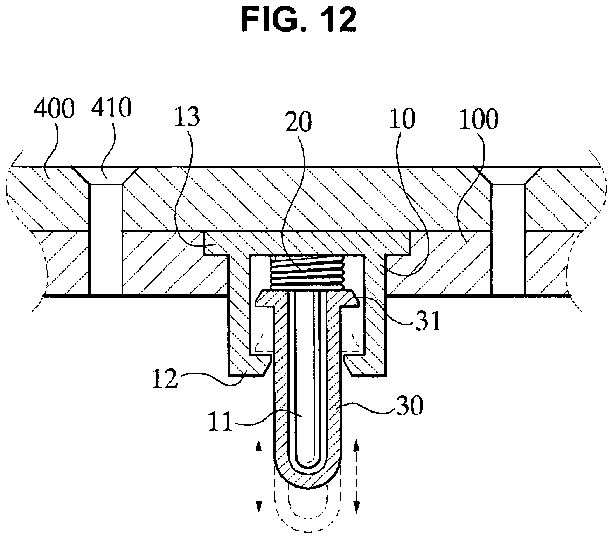

FIG. 12 is an enlarged view showing a hole of the cover of FIGS. 10 and 11.

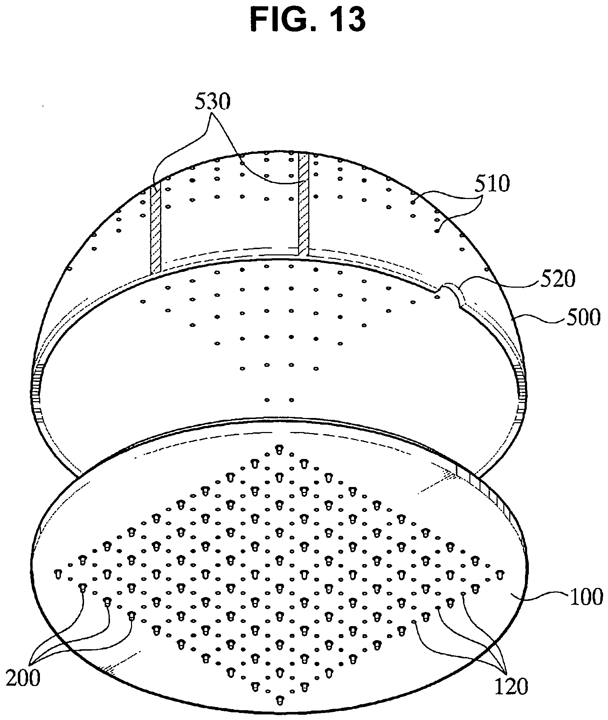

FIG. 13 is a perspective view showing the apparatus of FIG. 8 comprising another examplary cover.

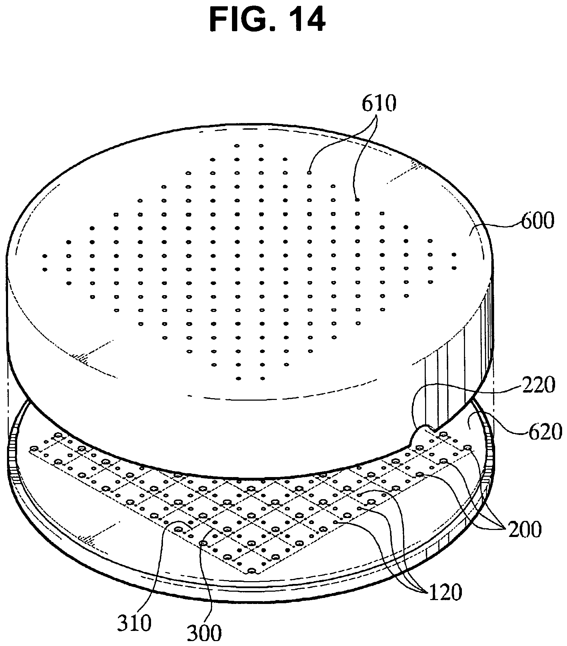

FIG. 14 is a perspective view showing the apparatus of FIG. 8 comprising a still another examplary cover.

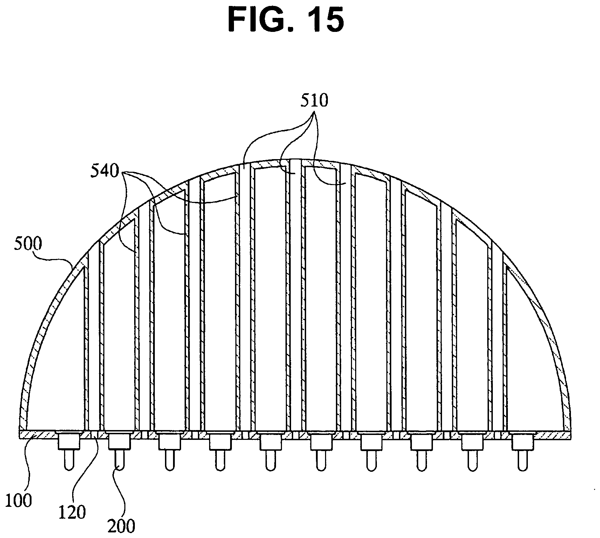

FIG. 15 is an enlarged view showing a guide hole member of the covers of FIG. 13 and 14.

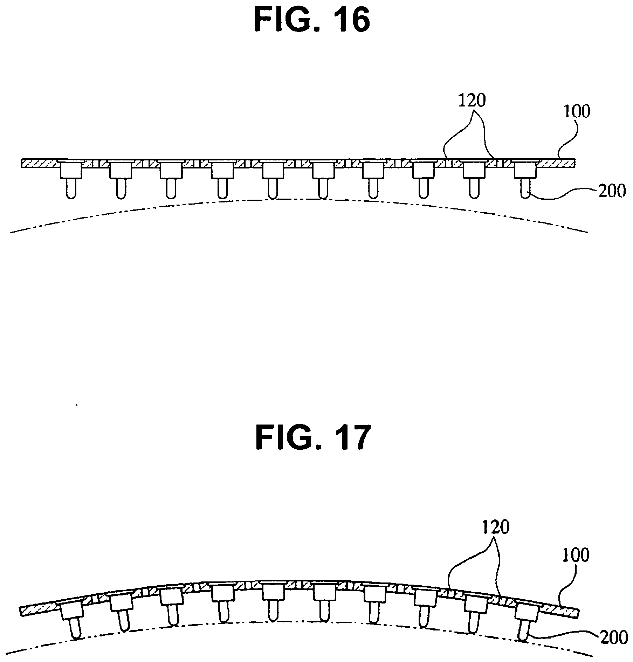

FIGS. 16 and 17 illustrate that an apparatus according to the present invention has flexibility.

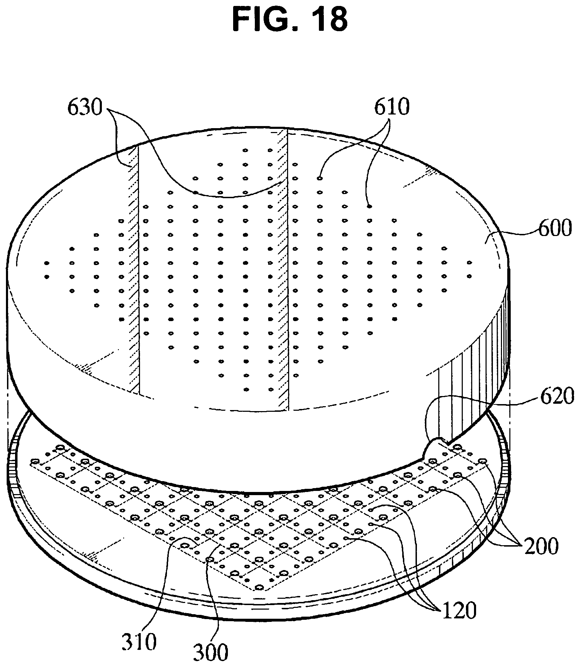

FIG. 18 is a perspective view showing the apparatus of FIG. 8 comprising a still further examplary cover.

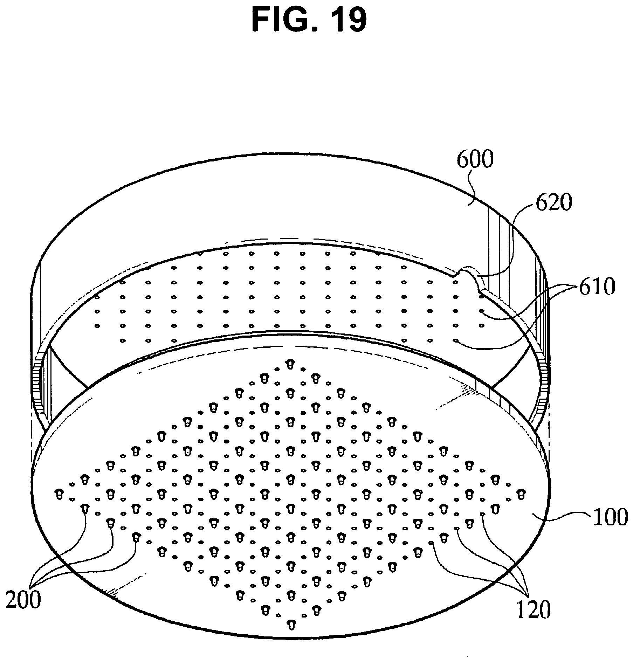

FIG. 19 is a perspective view showing the bottom of the apparatus of FIG. 18 comprising a still further examplary cover.

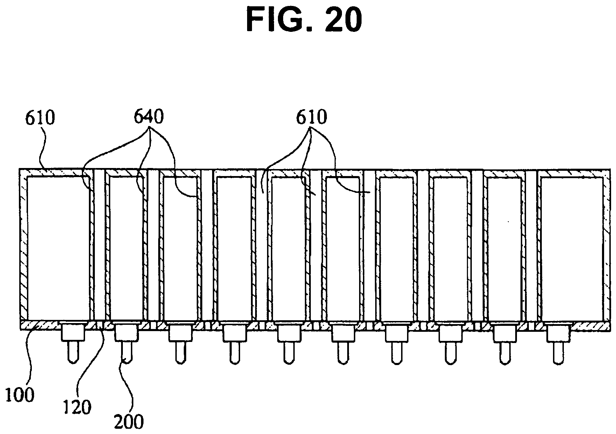

FIG. 20 is an enlarged view showing a guide hole member of the covers of FIG. 18 and 19.

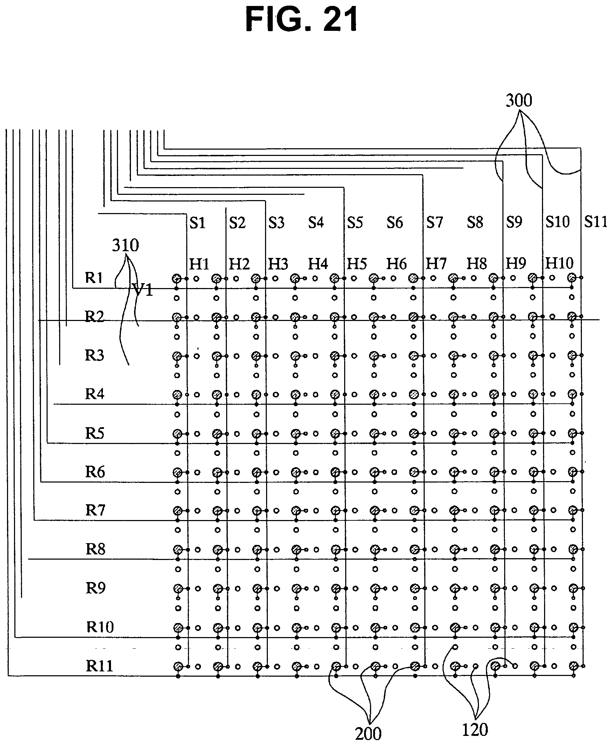

FIG. 21 shows electrode devices and electrical lines of an apparatus according to the first embodiment of the present invention.

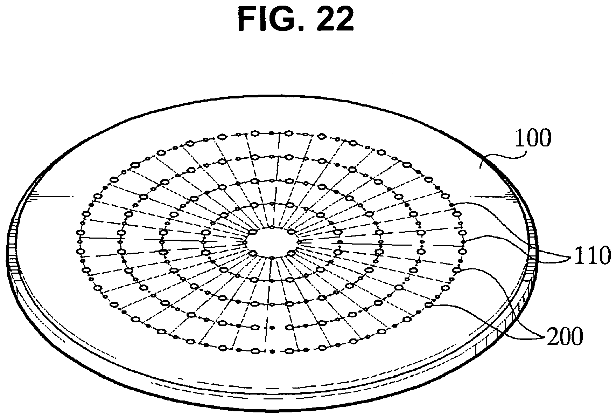

FIG. 22 is a perspective view of an apparatus for measuring impedance of a human body according to the second embodiment of the present invention.

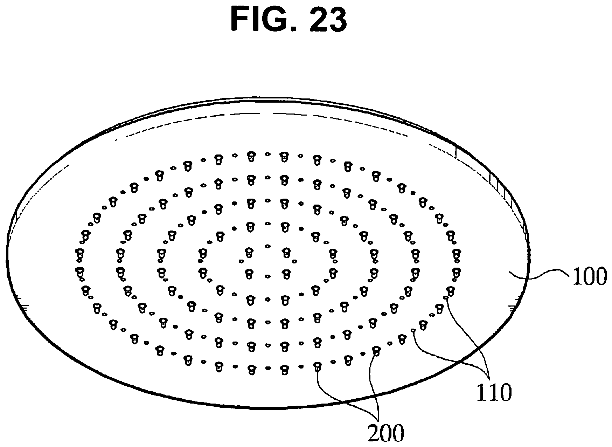

FIG. 23 is a perspective view of the bottom of the apparatus of FIG. 22.

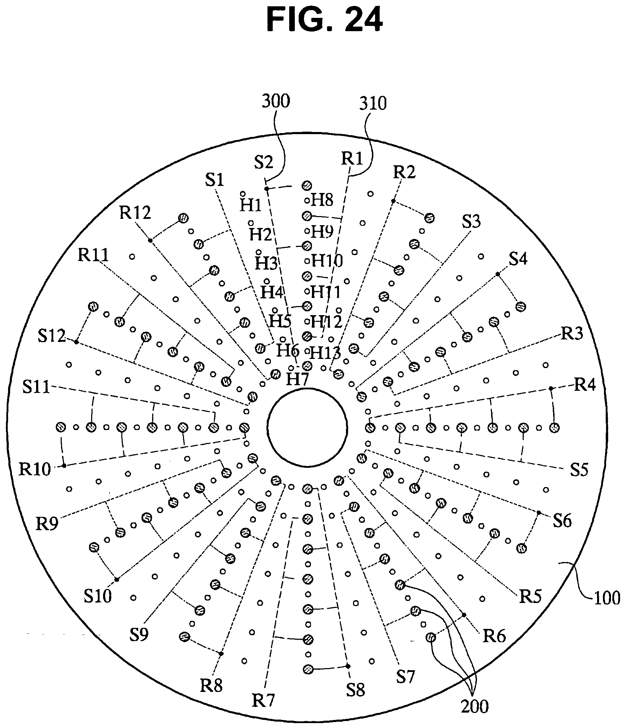

FIG. 24 shows electrode devices and electrical lines of the apparatus of FIG. 22.

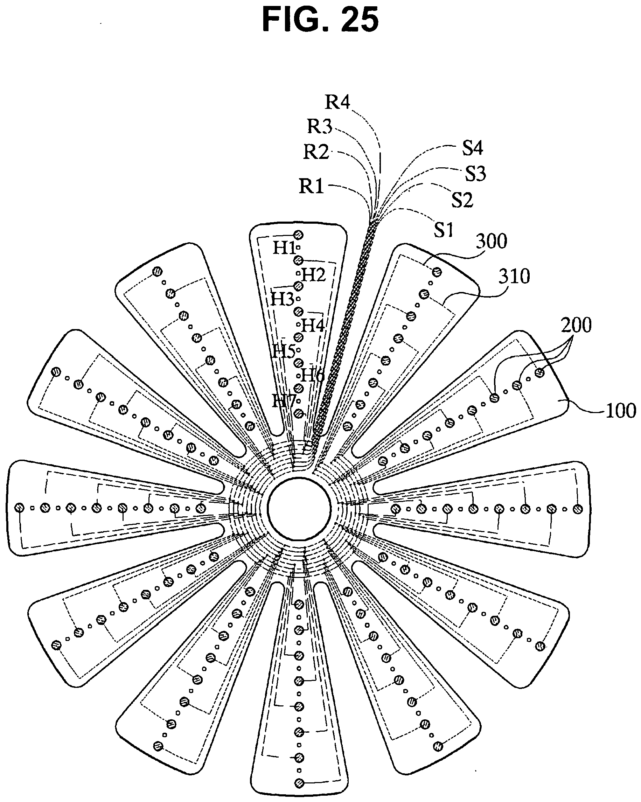

FIG. 25 is a plane view of an apparatus according the third embodiment of the present invention.

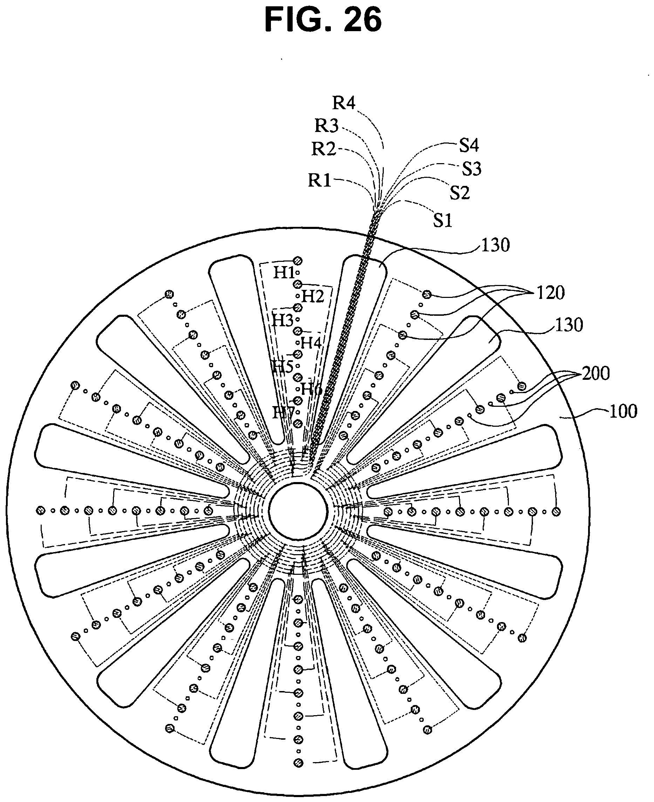

FIG. 26 is a plane view of an apparatus according the fourth embodiment of the present invention.

Reference numerals set forth in the Drawings includes reference to the following elements as further discussed below:

| | |
|---|---|
| 10: housing member | 11: guide rod |
| 20: resilient member | 30: cylindrical electrode member |
| 100: base plate | 110: electrode hole |
| 120: first needle hole | 130: radial hole |
| 200: electrode device | 300, 310: electrical lines |
| 400, 500, 600: cover | 410, 510, 610: second needle hole |
| 420, 520, 620: connecting hole | 430, 530, 630: identifying member |

### MODE OF THE INVENTION

Reference will now be made in detail to the preferred embodiment of the present invention, examples of which are illustrated in the drawings attached hereinafter, wherein like reference numerals refer to like elements throughout. The embodiments are described below so as to explain the present invention by referring to the figures.

Terms used herein are those used generally in the art unless otherwise being defined herein, in which case such terms are described in the specification and they should be construed by replying on the description. Also, detailed description of the subject matter that is known to those skilled in the art and is not directly related to the present invention is omitted for simplicity.

FIG. 5 is a cross-sectional view showing a dissembled state of an electrode device for measuring impedance of a human body according to the first embodiment of the present invention and FIG. 6 is a cross-sectional view showing an assembled state of the electrode device of FIG. 5.

The electrode device according to the first embodiment, as shown in FIGS. 5 and 6, comprises a cylindrical housing member (10), a cylindrical electrode member (20), and a resilient member (30). The cylindrical housing member (10) has a guide rod (11) mounted to an open side thereof. The cylindrical electrode member (30) is configured to be pressed against the skin of a human body to move back and forth through the open side along the guide rod (11). The resilient member (30) is interposed between the cylindrical electrode member (20) and the cylindrical housing member (10) to resiliently move the cylindrical electrode member (20) back and forth.

The cylindrical electrode member (30) can be an electrode made of a conductive material. It can also be an electrode coated with a conductive material. Any material that shows conductivity can be used as the conductive material. Preferably, the conductive material is non-toxic to a human body. As an example, an electrode made of a gold or an electrode coated with gold can be used. The cylindrical electrode member (30) has an open end. A protruding portion (31) is formed at the open end of the cylindrical electrode member (30). The protruding portion (31) may be formed in any shape that can facilitate engagement between the cylindrical electrode member (30) and the cylindrical housing member (10).

The cylindrical housing member (10) can be made of a conductive material. It can also be an electrode coated with a conductive material. Any material that shows conductivity can be used as the conductive material. As an example, an electrode made of gold or an electrode coated with gold can be used. Also, a copper wire or an iron wire can be used. A first protruding portion (12) is formed at the open side of the cylindrical housing member (10). The first housing protruding portion (12) is formed in any shape that can facilitate engagement between the cylindrical electrode member (30) and the cylindrical housing member (10). A second housing portion (13) is formed at the other side of the cylindrical housing member (10).

The resilient member (20) can be made of any material that can show resilient features. The resilient material can also be a conductive material. As an example, a spring can be used.

FIG. 7 is a cross-sectional view showing an example of application of the electrode device of FIG. 5. As shown in FIG. 7, when the cylindrical electrode member (30) is pressed against the skin of a human body, the cylindrical electrode member (30) can be moved along the guide rod by the resilient member (20) according to the curvature of the skin. Accordingly, the depths of the cylindrical electrode members that are attached to the skin can be set to be a certain value. This can avoid a prior art problem that impedance was not be able to be measured precisely since the depths of electrode devices attached to the skin are not constant. The reference number 100 refers to a base plate in which electrode devices according to the present invention are mounted.

FIG. 8 is a perspective view of an apparatus for measuring impedance of a human body according to a first embodiment of the present invention, and FIG. 9 is a perspective view of the bottom of the apparatus of FIG. 8. As shown in FIGS. 8 and 9, the apparatus according to the first embodiment comprises a base plate (100) and a plurality of electrode devices (200). The base plate (100) has a plurality of electrode holes (110) and a plurality of first needle holes (120). The electrode holes (100) are arranged in an X-Y lattice structure. The first needle holes (120) are arranged in an X-Y lattice structure such that they are arranged with the electrode holes (100) alternately. The electrode devices each are mounted in the respective electrode holes (110). The electrode devices are connected to a plurality of first and second electrical lines (300, 310), which will be described in detail with reference to FIG. 21. The base plate (100) can be made of any soft material that can make the base plate curved according to the curvature of the skin of a human body. As an example, it can be made of silicone.

The distance between two adjacent electrode holes and the distance between two adjacent first needle holes are not limited to a specific value. Preferably, the distances can be set in a range of 5mm to 20mm.

The width of the first needle holes each is not limited to a specific value. It can be appropriately set according to the kind of a needle and/or the purpose of acupuncture.

FIG. 10 is a perspective view showing the apparatus of FIG. 8 comprising an examplary cover and FIG. 11 is a perspective view showing the bottom of the apparatus comprising the cover. The exemplary cover (400), as shown in FIGS. 10 and 11, is configured to be engaged with the base plate (100) to protect the electrode devices (200) and the first and second electrical lines (300, 310). The cover (400) has a plurality of second needle holes (410). The second needle holes (410) are arranged in the cover (400) such that the respective second needle holes (410) can be positioned over the respective first needle holes (120) of the base plate (100) when the cover (400) is engaged with the base plate (100). The second needle holes can be designed to have the same width as the first needle holes. Preferably, as shown in FIG. 12, the second needle holes (410) can be designed such that the ourter width is greater than the inner width, which makes it easier for a user to insert a needle to the second needle holes (410). In some embodiments, at least one identifying member (430) may be provided on the cover (400) to make it easier for a user to handle the cover (400). The cover (400) can be made of any material that can perform the above-described function. As an example, it can be made of silicone. The cover (400) is provided with a connecting hole (420) through which electrical lines can be connected.

FIG. 13 is perspective view showing the apparatus of FIG. 8 comprising another examplary cover. As shown in FIG. 13, the cover (500) is in the form of a hemisphere. The cover (500) has a plurality of second needle holes (510). The second needle holes (510) are arranged in the cover (500) such that the respective second needle holes (510) can be positioned over the respective first needle holes (120) of the base plate (100) when the cover (500) is engaged with the base plate (100). In some embodiments, the base plate (100) is designed to be flexible so as to become curved according to the curvature of the skin, as shown in FIG. 17 while the base plate (100) is flat in normal condition, as shown in FIG. 16. In some embodiments, as shown in FIG. 15, the cover (500) has a plurality of guide hole members (540) each connecting the first needle holes (120) and the second needle holes (510) to make it easier for a user to handle a needle. As described above, preferably, the second needle holes (510) can be designed such that the outer width is greater than the inner width, which makes it easier for a user to insert a needle to the second needle holes (510). The cover (500) can be made of any material that can perform the above-described function. As an example, it can be made of silicone. The cover (500) is provided with a connecting hole (520) through which electrical lines can be connected. In some embodiments, at least one identifying member (530) may be provided on the cover (500) to make it easier for a user to handle the cover (500).

FIGS. 14 and 18 are perspective views showing the apparatus of FIG. 8 comprising another examplary cover and FIG. 19 is a perspective view showing the bottom of the apparatus comprising the cover shown in FIG. 18. As shown in the drawings, the cover (600) is in the form of a cylinder. The cover (600) has a plurality of second needle holes (610). The second needle holes (610) are arranged in the cover (600) such that the respective second needle holes (610) can be positioned over the respective first needle holes (120) of the base plate (100) when the cover (600) is engaged with the base plate (100). In some embodiments, the base plate (100) is designed to be flexible so as to become curved according to the curvature of the skin, as shown in FIG. 17 while the base plate (100) is flat in normal condition, as shown in FIG. 16. In some embodiments, as shown in FIG. 20, the cover (500) has a plurality of guide hole members (640) connecting the first needle holes (120) and the second needle holes (610) to make it easier for a user to handle a needle. As described above, preferably, the second needle holes (610) can be designed such that the outer width is greater than the inner width, which makes it easier for a user to insert a needle to the second needle holes (610). The cover (600) can be made of any material that can perform the above-described function. As an example, it can be made of silicone. The cover (600) is provided with a connecting hole (620) through which electrical lines can be connected. In some embodiments, at least one identifying member (630) may be provided on the cover (600) to make it easier for a user to handle the cover (600).

FIG. 21 shows electrode devices and electrical lines of an apparatus according to the first embodiment of the present invention. The electrode devices (200) and the first needle holes (120) are positioned on the base plate (100) such that they are alternating with each other. The first electrical lines (300) are formed between the respective electrode devices (200) and the respective first needle holes (120) in the direction of X-axis. The second electrical lines (310) are formed between the respective electrode devices (200) and the respective first needle holes (120) in the direction of Y-axis.

The respective first and second electrical lines (300, 310) are connected to the respective electrode devices (200). Current is allowed to flow through the X-axis and Y-axis sequentially to measure impedances. For example, with the second vertical line S2 as an input electrode and the first horizontal line R1 as a receiving electrode, the impedance of the first horizontal needle hole H1 can be measured. Also, with the first vertical line S1 as an input electrode and the second horizontal line R2 as a receiving electrode, the impedance of the first vertical needle hole V1 can be measured. Accordingly, if current is allowed to flow from the first vertical line S1 to the eleventh vertical line S11 sequentially to act as input electrodes and from the horizontal line R1 to the eleventh horizontal line R11 to act as receiving electrodes, impedances can be measured sequentially with regard to all of the vertical and horizontal needle holes of the base plate (100). The measured impedances can be displayed as a 3-dimensional image by being coupled with CT or MRI devices.

FIG. 22 is a perspective view of an apparatus according to the second embodiment of the present invention and FIG. 23 is a perspective view of the bottom of the apparatus of FIG. 22. As shown in FIGS. 22 and 23, the apparatus according to the second embodiment comprises a circular base plate (100) and a plurality of electrode devices (200). The circular base plate (100) includes a plurality of electrode hoes (110) and a plurality of first needle holes (120). The electrode holes (110) and first needle holes (120) are formed eccentrically and alternately, as shown in FIG. 22. The electrode devices (200) are mounted to the electrode holes (110) and connected to a first and second electrical lines (300, 310)

FIG. 24 shows electrode devices and electrical lines of the apparatus of FIG. 22. The first and second electrical lines (300, 310) are connected to the electrode devices alternately. Current is allowed sequentially to measure impedances. For example, with the second input line S2 as an input electrode and the twelveth receiving line R12 as a receiving electrode, the impedances of the first, third, fifth, and seventh needle holes H1, H3, H5, and H5 can be measured. Also, with the first input line S1 as an input electrode and the first receiving line R1 as a receiving electrode, the impedances of the second, fourth, and sixth needle holes H2, H4, and H6 can be measured. Accordingly, if current is allowed to flow from the first input line S1 to the twelveth input line S12 and from the first receiving line R1 to the twelveth receiving line R12 sequentially to act as input electrodes and receiving electrodes, respectively, the impedances can be measured sequentially with regard to all of needle holes of the base plate (100).

FIG. 25 is a plane view of an apparatus according the third embodiment of the present invention. In this embodiment, a base plate (100) has multiple parts that are provided radially with a predetermined distance between two adjacent parts. Each of the parts has a plurality of electrode holes and a plurality of first needle holes (120). The electrode holes and the first needle holes are mounted alternately. Current is allowed sequentially to measure impedances. For example, with the first input line S1 as an input electrode and the first receiving line R1 as a receiving electrode, the impedance of the first needle hole H1 can be measured. Also, with the second input line S2 as an input electrode and the first receiving line R1 as a receiving electrode, the impedance of the second needle hole H2 can be measured. Accordingly, if current is allowed to flow sequentially, the impedances can be measured sequentially with regard to all of needle holes of the base plate (100).

FIG. 26 is a plane view of an apparatus according the fourth embodiment of the present invention. In this embodiment, the apparatus comprises a base plate, a plurality of electrode devices, and first and second electrical lines. The base plate has a plurality of radial holes provided radially with a predetermined distance between two adjacent holes. The base plate has a plurality of electrode holes and a plurality of first needle holes between two adjacent radial holes. The electrode holes and the first needle holes are mounted alternately. The electrode devices are mounted to the electrode holes. The first and second electrical lines are connected to the electrode devices alternately. As the principle of measuring impedances is similar to the principle described with regard to FIG. 25, detailed description thereof is omitted.

The foregoing descriptions of specific exemplary embodiments of the present invention have been presented for purposes of illustration and description. They are not intended to be exhaustive or to limit the invention to the precise forms disclosed, and obviously many modifications and variations are possible in light of the above teachings. The exemplary embodiments were chosen and described in order to explain certain principles of the invention and their practical application, to thereby enable others skilled in the art to make and utilize various exemplary embodiments of the present invention, as well as various alternatives and modifications thereof. It is intended that the scope of the invention be defined by the Claims appended hereto.

## Claims

1. An electrode device (200) for measuring impedances of a human body, comprising:
- a housing member (10) having an open side;
- a guide rod (11) that has first and second ends, that extends from an inner surface of the cylindrical housing member (10) such that the first end of the guide rod (11) is disposed at the inner surface, and that extends through the open side of the cylindrical housing member (10);
- a cylindrical electrode member (30) having an open end and configured to be pressed against the skin of a human body to move back and forth through the open side of the cylindrical housing member (10) along the guide rod (11);
the cylindrical electrode member (30) being disposed around the guide rod (11) and the cylindrical electrode member (30) having a protruding portion (31) formed at the open end thereof that allows the cylindrical electrode member (30) to engage with the cylindrical housing member (10); and
- a spring (20) surrounding the guide rod (11) and interposed between the top of the cylindrical electrode member (30) and the top of the cylindrical housing member (10) to resiliently move the cylindrical electrode member (10) back and forth along the guide rod (11), the electrode device **characterised in that**:
- the housing member is a cylindrical housing member
- the spring (20) abuts against the protruding portion (31) at the open end of the cylindrical electrode member (30)
- a first housing protruding portion (12) is formed at the open side of said cylindrical housing member (10) such that engagement between the cylindrical electrode member (30) and said cylindrical housing member (10) is facilitated, and
- a second housing protruding portion (13) is formed at an other side of said cylindrical housing member (10) such that engagement between said cylindrical housing member (10) and a base plate (100) is facilitated.

2. The electrode device (200) of claim 1,
- wherein the cylindrical electrode member (30) is an electrode made of a conductive material or an electrode coated with a conductive material,
- wherein in particular the conductive material is gold.

3. The electrode device (200) of claim 1,
wherein the cylindrical housing member (10) is made of a conductive material or an electrode coated with a conductive material.

4. The electrode device (200) of claim 1,
wherein said first housing protruding portion (12) is formed at the open side of the cylindrical housing member (10) and said second housing protruding portion (13) is formed at the other side of the cylindrical housing member (10).

5. The electrode device (200) of claim 1,
wherein the resilient member (20) is made of a conductive material.

6. An apparatus for measuring impedance of a human body, which comprises:
- a base plate (100) having a plurality of electrode holes (200) formed in a lattice structure and a plurality of a first needle holes (120) formed in a lattice structure such that the electrode holes (200) and the first needle holes (120) are arranged alternately;
- a plurality of electrode devices according to any of the preceding claims mounted to the electrode holes; and
- first and second electrical lines (300, 310) connected to the electrode devices.

7. The apparatus of claim 6,
wherein the distance between two adjacent electrode holes (200) and the distance between two adjacent first needle holes (120) are set in a range of 5 mm to 20 mm.

8. The apparatus of claim 6,
further comprising a cover (400; 500) configured to be engaged with the base plate (100) to protect the electrode devices and the first and second electrical lines (300, 310).

9. The apparatus of claim 7,
wherein the cover includes a plurality of second needle holes (410) arranged such that the respective second needle holes (410) can be positioned over the respective first needle holes (120) of the base plate (100) when the cover (400; 500) is engaged with the base plate (100).

10. The apparatus of claim 9,
wherein the outer width of the second needle holes (410) is greater than the inner width thereof.

11. The apparatus of claim 8,
- wherein the cover (400; 500) is provided with at least one identifying member and/or
- wherein the cover (400; 500) is made of silicone and/or
- wherein the cover (400; 500) is shaped to be a hemisphere or a cylinder.

12. The apparatus of claim 11,
wherein the cover (500) further comprises a plurality of guide hole members (540) each connecting the first needle holes (120) and the second needle holes (400).

13. The apparatus of claim 6,
wherein the first electrical lines (300) are formed between the respective electrode devices and the respective first needle holes in the direction of X-axis and the second electrical lines (310) are formed between the respective electrode devices and the respective first needle holes in the direction of Y-axis.

14. The apparatus of claim 6,
wherein the first electrical lines (300) are connected to input electrodes and the second electrical lines (310) are connected to receiving electrodes.

15. An apparatus for measuring impedances of a human body, comprising:
- a circular base plate (100) including a plurality of electrode holes (110) and a plurality of first needle holes (120), the electrode holes (110) and the first needle holes (120) being formed eccentrically and alternately;
- a plurality of electrode devices (200) according to any of claims 1 to 5 mounted to the electrode holes (110); and
- first and second electrical lines (300, 310) connected to the electrode devices (200).

## Patentansprüche

1. Elektrodengerät (200) zum Messen von Impedanzen eines menschlichen Körpers, umfassend:
- ein Gehäuseelement (10) mit einer offenen Seite;
- eine Führungsstange (11), die ein erstes und zweites Ende umfasst, die sich von einer inneren Oberfläche des zylindrischen Gehäuseelements (10) erstreckt, so dass das erste Ende der Führungsstange (11) an der inneren Oberfläche angeordnet ist, und die sich durch die offene Seite des zylindrischen Gehäuseelements (10) erstreckt;
- ein zylindrisches Elektrodenelement (30) mit einer offenen Seite, das eingerichtet ist, dass es gegen die Haut eines menschlichen Körpers gedrückt ist, um sich durch die offene Seite des zylindrischen Gehäuseelements (10) entlang der Führungsstange (11) hin und herzu bewegen,
wobei das zylindrische Elektrodenelement (30) um die Führungsstange (11) herum angeordnet ist, und das zylindrische Elektrodenelement (30) mit einem vorstehenden Bereich (31), der am offenen Ende davon ausgebildet ist, der zulässt, dass das zylindrische Elektronenelement (30) mit dem zylindrischen Gehäuseelement (10) in Eingriff kommt; und
- eine Feder (20), die die Führungsstange (11) umgibt und zwischen der Spitze des zylindrischen Elektrodenelements (30) und der Spitze des zylindrischen Gehäuseelements (10) eingefügt ist, um das zylindrische Elektrodenelement (10) entlang der Führungsstange (11) hin und her elastisch zu bewegen,
wobei das Elektrodengerät **dadurch gekennzeichnet ist, dass**:
- das Gehäuseelement ein zylindrisches Gehäuseelement ist;
- die Feder (20) gegen den vorstehenden Bereich (31) am offenen Ende des zylindrischen Elektrodenelements (30) anstößt;
- ein erster vorstehender Gehäusebereich (12) an der offenen Seite des zylindrischen Gehäuseelements ausgebildet ist, sodass das in Eingriff Kommen zwischen dem zylindrischen Elektrodenelement (30) und dem zylindrischen Gehäuseelement (10) vereinfacht ist, und
- ein zweiter vorstehender Gehäusebereich (13) an einer anderen Seite des zylindrischen Gehäuseelements (10) ausgebildet ist, sodass das in Eingriff Kommen zwischen dem zylindrischen Gehäuseelement (10) und einer Basisplatte (100) vereinfacht ist.

2. Das Elektrodengerät (200) nach Anspruch 1,
- wobei das zylindrische Elektrodenelement (30) entweder eine Elektrode, die aus einem leitenden Material, oder eine Elektrode, die mit einem leitenden Material bedeckt ist, ist,
- wobei das leitende Material insbesondere Gold ist.

3. Das Elektrodengerät (200) nach Anspruch 1,
wobei das zylindrische Gehäuseelement (10) entweder eine Elektrode, die aus einem leitenden Material, oder eine Elektrode, die mit einem leitenden Material bedeckt ist, ist.

4. Das Elektrodengerät (200) nach Anspruch 1,
wobei der genannte erste vorstehende Gehäusebereich (12) an der offenen Seite des zylindrischen Gehäuseelements (10) ausgebildet ist, und der genannte zweite vorstehende Gehäusebereich (13) an der anderen Seite des zylindrischen Gehäuseelements (10) ausgebildet ist.

5. Das Elektrodengerät (200) nach Anspruch 1,
wobei das elastische Element (20) aus einem leitenden Material aufgebaut ist.

6. Eine Vorrichtung zum Messen von Impedanz eines menschlichen Körpers, die umfasst:
- eine Basisplatte (100) mit einer Vielzahl von Elektronenlöchern (200), die in einer Gitterstruktur gebildet sind, und einer Vielzahl von ersten Nadellöchern (120), die in einer Gitterstruktur ausgebildet sind, sodass die Elektrodenlöcher (200) und die ersten Nadellöcher (120) abwechselnd eingerichtet sind;
- eine Vielzahl von Elektrodengeräten nach einem der vorangehenden Ansprüche, die an den Elektrodenlöchern montiert sind; und
- erste und zweite elektrische Leitungen (300, 310), die an den Elektrodengeräten verbunden sind.

7. Die Vorrichtung nach Anspruch 6,
wobei der Abstand zwischen zwei benachbarten Elektrodenlöchern (200) und der Abstand zwischen zwei benachbarten ersten Nadellöchern (120) in einem Bereich von 5 mm bis 20 mm gesetzt sind.

8. Die Vorrichtung nach Anspruch 6,
ferner umfassend eine Abdeckung (400, 500), die eingerichtet ist, mit der Basisplatte (100) in Eingriff zu kommen, um die Elektrodengeräte und die ersten und zweiten elektrischen Leitungen (300, 310) zu schützen.

9. Die Vorrichtung nach Anspruch 7,
wobei die Abdeckung eine Vielzahl von zweiten Nadellöchern (410) umfasst, die angeordnet sind, sodass die jeweiligen zweiten Nadellöcher (410) über die jeweiligen ersten Nadellöcher (120) der Basisplatte (100) positioniert werden können, wenn sich die Abdeckung (400; 500) mit der Basisplatte (100) in Eingriff befindet.

10. Die Vorrichtung nach Anspruch 9,
wobei die äußere Breite der zweiten Nadellöcher (410) größer als die innere Breite davon.

11. Die Vorrichtung nach Anspruch 8,
- wobei die Abdeckung (400; 500) mit zumindest einem Identifikationselement versehen ist, und/oder
- wobei die Abdeckung (400; 500) aus Silikon ist, und/oder
- wobei die Abdeckung (400; 500) ausgebildet ist, um eine Hemisphäre oder ein Zylinder zu sein.

12. Die Vorrichtung nach Anspruch 11,
wobei die Abdeckung (500) weiter eine Vielzahl von Führungslochelementen (540) umfasst, jedes Einzelne davon die ersten Nadellöcher (120) und die zweiten Nadellöcher (400) miteinander verbindet.

13. Die Vorrichtung nach Anspruch 6,
wobei die ersten elektrischen Leitungen (300) zwischen den jeweiligen Elektrodengeräten und den jeweiligen ersten Nadellöchern in der Richtung der X-Achse ausgebildet sind, und die zweiten elektrischen Leitungen (310) zwischen den jeweiligen Elektrodengeräten und den jeweiligen ersten Nadellöchern in der Richtung der Y-Achse ausgebildet sind.

14. Die Vorrichtung nach Anspruch 6,
wobei die ersten elektrischen Leitungen (300) mit Eingabeelektroden verbunden sind, und die zweiten elektrischen Leitungen (310) mit Empfangselektroden verbunden sind.

15. Die Vorrichtung zum Messen von Impedanzen eines menschlichen Körpers, umfassend:
- eine kreisförmige Basisplatte (100) umfassend eine Vielzahl von Elektrodenlöchern (110) und eine Vielzahl von ersten Nadellöchern (120), wobei die Elektrodenlöcher (110) und die ersten Nadellöcher (120) exzentrisch und abwechselnd ausgebildet sind;
- eine Vielzahl von Elektrodengeräten (200) nach einem der Ansprüche 1 bis 5, die an den Elektrodenlöcher (110) montiert sind, und
- erste und zweite elektrische Leitungen (300, 310), die mit den Elektrodengeräten (200) verbunden sind.

## Revendications

1. Dispositif d'électrode (200) pour mesurer des impédances d'un corps humain, comprenant :
- un élément de logement (10) ayant un côté ouvert ;
- une tige de guidage (11) qui a des première et deuxième extrémités, qui s'étend depuis une surface interne de l'élément de logement cylindrique (10) de sorte que la première extrémité de la tige de guidage (11) soit disposée au niveau de la surface interne, et qui s'étend à travers le côté ouvert de l'élément de logement cylindrique (10) ;
- un élément d'électrode cylindrique (30) ayant une extrémité ouverte et configuré pour être pressé contre la peau d'un corps humain afin d'effectuer un mouvement de va-et-vient à travers le côté ouvert de l'élément de logement cylindrique (10) le long de la tige de guidage (11);
l'élément d'électrode cylindrique (30) étant disposé autour de la tige de guidage (11) et l'élément d'électrode cylindrique (30) ayant une partie en saillie (31) formée au niveau de son extrémité ouverte qui permet à l'élément d'électrode cylindrique (30) de s'engager avec l'élément de logement cylindrique (10) ; et
- un ressort (20) entourant la tige de guidage (11) et interposé entre la partie supérieure de l'élément d'électrode cylindrique (30) et la partie supérieure de l'élément de logement cylindrique (10) pour amener l'élément d'électrode cylindrique (10) à effectuer, de manière élastique, un mouvement de va-et-vient le long de la tige de guidage (11),
le dispositif d'électrode étant **caractérisé en ce que** :
- l'élément de logement est un élément de logement cylindrique ;
- le ressort (20) vient en butée contre la partie en saillie (31) au niveau de l'extrémité ouverte de l'élément d'électrode cylindrique (30) ;
- une première partie en saillie de logement (12) est formée sur le côté ouvert dudit élément de logement cylindrique (10) de sorte que l'engagement entre l'élément d'électrode cylindrique (30) et ledit élément de logement cylindrique (10) soit facilité, et
- une deuxième partie en saillie de logement (13) est formée sur un autre côté dudit élément de logement cylindrique (10) de sorte que l'engagement entre ledit élément de logement cylindrique (10) et une plaque de base (100) soit facilité.

2. Dispositif d'électrode (200) de la revendication 1,
- dans lequel l'élément d'électrode cylindrique (30) est une électrode réalisée en un matériau conducteur ou une électrode revêtue d'un matériau conducteur,
- dans lequel, en particulier, le matériau conducteur est de l'or.

3. Dispositif d'électrode (200) de la revendication 1,
dans lequel l'élément de logement cylindrique (10) est réalisé en un matériau conducteur ou est une électrode revêtue d'un matériau conducteur.

4. Dispositif d'électrode (200) de la revendication 1,
dans lequel ladite première partie en saillie de logement (12) est formée sur le côté ouvert de l'élément de logement cylindrique (10) et ladite deuxième partie en saillie de logement (13) est formée sur l'autre côté de l'élément de logement cylindrique (10).

5. Dispositif d'électrode (200) de la revendication 1,
dans lequel l'élément élastique (20) est réalisé en un matériau conducteur.

6. Appareil pour mesurer l'impédance d'un corps humain, qui comprend :
- une plaque de base (100) ayant une pluralité de trous d'électrodes (200) formée selon une structure en treillis et une pluralité de premiers trous d'aiguille (120) formée selon une structure en treillis de sorte que les trous d'électrodes (200) et les premiers trous d'aiguille (120) soient agencés alternativement ;
- une pluralité de dispositifs d'électrodes selon l'une des revendications précédentes, montés sur les trous d'électrodes ; et
- des premières et deuxièmes lignes électriques (300, 310) reliées aux dispositifs d'électrodes.

7. Appareil de la revendication 6,
dans lequel la distance entre deux trous d'électrodes adjacents (200) et la distance entre deux premiers trous d'aiguille adjacents (120) sont définies dans une plage allant de 5 mm à 20 mm.

8. Appareil de la revendication 6,
comprenant en outre un couvercle (400 ; 500) configuré pour être engagé avec la plaque de base (100) afin de protéger les dispositifs d'électrodes et les premières et deuxièmes lignes électriques (300, 310).

9. Appareil de la revendication 7,
dans lequel le couvercle comporte une pluralité de deuxièmes trous d'aiguille (410) agencée de sorte que les deuxièmes trous d'aiguille respectifs (410) puissent être positionnés sur les premiers trous d'aiguille respectifs (120) de la plaque de base (100) lorsque le couvercle (400 ; 500) est engagé avec la plaque de base (100).

10. Appareil de la revendication 9,
dans lequel la largeur externe des deuxièmes trous d'aiguille (410) est supérieure à la largeur interne de ceux-ci.

11. Appareil de la revendication 8,
- dans lequel le couvercle (400 ; 500) est doté d'au moins un élément d'identification et / ou
- dans lequel le couvercle (400 ; 500) est réalisé en silicone et / ou
- dans lequel le couvercle (400 ; 500) est façonné pour être un hémisphère ou un cylindre.

12. Appareil de la revendication 11,
dans lequel le couvercle (500) comprend en outre une pluralité d'éléments de trou de guidage (540) reliant chacun les premiers trous d'aiguille (120) et les deuxièmes trous d'aiguille (400).

13. Appareil de la revendication 6,
dans lequel les premières lignes électriques (300) sont formées entre les dispositifs d'électrodes respectifs et les premiers trous d'aiguille respectifs selon la direction de l'axe des X et les deuxièmes lignes électriques (310) sont formées entre les dispositifs d'électrodes respectifs et les premiers trous d'aiguille respectifs selon la direction de l'axe des Y.

14. Appareil de la revendication 6,
dans lequel les premières lignes électriques (300) sont reliées à des électrodes d'entrée et les deuxièmes lignes électriques (310) sont reliées à des électrodes de réception.

15. Appareil pour mesurer des impédances d'un corps humain, comprenant :
- une plaque de base circulaire (100) comportant une pluralité de trous d'électrodes (110) et une pluralité de premiers trous d'aiguille (120), les trous d'électrodes (110) et les premiers trous d'aiguille (120) étant formés de manière excentrique et alternée ;
- une pluralité de dispositifs d'électrodes (200) selon l'une des revendications 1 à 5 montés sur les trous d'électrodes (110) ; et
- des premières et deuxièmes lignes électriques (300, 310) reliées aux dispositifs d'électrodes (200).
